# EUROPÄISCHE PATENTANMELDUNG

(11) **EP 4 252 796 A1**
(43) Veröffentlichungstag der Anmeldung: **04.10.2023**
(21) Anmeldenummer: 22166105.1
(22) Anmeldetag: 31.03.2022
(51) Int. Cl.: A61M 5/14, H01R 29/00, H01H 3/00, G01D 5/25, G06K 19/067, A61M 5/142, H02J 7/00, A61M 39/10

(54) **GERÄT ZUM BETRIEB EINER MEDIZINISCHEN PUMPE**

(71) Anmelder: Erbe Elektromedizin GmbH, 72072 Tübingen (DE)
(72) Erfinder: MUELLER, Kilian, 72108 Rottenburg (DE); MASKOS, Isabel, 72116 Moessingen (DE)
(74) Vertreter: Rüger Abel Patentanwälte PartGmbB

(57) **Zusammenfassung**

Das erfindungsgemäße Konzept richtet sich auf eine Pumpenantriebseinheit (15) und die zugehörige Pumpe (17), die einen Sitz (29) aufweist, der alternativ zur Aufnahme eines Speicherchips (31) oder von Kennkontaktträgern (34, 39) genutzt werden kann. Die Pumpenantriebseinheit weist mindestens vier elektrische Kontakte auf, die mit dem Kontaktträger (30) (entweder Speicherchip (31) oder Kennkontaktträger (34, 39)) bzw. deren Kontaktflächen (32, 33, 36, 37, 42, 43) in Kontakt überführbar sind. Eine in dem Pumpenantriebsgerät (15) vorgesehene Logikeinheit (55) kann dazu dienen zu unterscheiden, ob als Kontaktträger (30) ein Speicherchip (31) oder Kennkontaktträger (34, 39) angeordnet ist. Detektiert sie einen Speicherchip (31), tritt sie mit diesem in elektrische Kommunikation. Detektiert sie hingegen einen Kennkontaktträger (34, 39) bestimmt sie anhand der Ausrichtung seiner Kontakteflächen (32,33; 36,37; 42,43) dessen Typ und gibt ein entsprechendes pumpentypkennzeichnendes Signal ab.

Das erfindungsgemäße Konzept eröffnet einen weiten Bereich von zusätzlichen Nutzungsmöglichkeiten grundsätzlich bekannter Pumpensysteme mit Nutzen für die Erhöhung der Patientensicherheit und Erleichterung der Bedienbarkeit.

## Beschreibung

Gegenstand der Erfindung ist ein Pumpenantriebsgerät insbesondere zum Antrieb von Kolbenpumpen. Außerdem richtet sich die Erfindung auf eine in das Pumpenantriebsgerät einsetzbare Pumpe.

Medizinische Pumpen sind zu verschiedenen Anwendungszwecken aus dem Stand der Technik bekannt. Beispielsweise offenbart die EP 2 088 942 B1 eine medizinische Pumpe, bestehend aus einer Pumpeneinheit und einer Pumpenantriebseinrichtung. Das korrekte Einsetzen der Pumpeneinheit in die Pumpenbetätigungseinrichtung wird beispielsweise mittels einer Lichtschranke erfasst. Der Pumpenbetrieb wird nur dann freigegeben, wenn die Lichtschranke oder andere Sensoren das Ankoppeln der Pumpeneinheit an der Pumpenbetätigungseinrichtung signalisieren.

Die DE 601 17 941 T2 offenbart eine Ultraschallbehandlungseinrichtung, bestehend aus einem Ultraschallhandstück und einem Treibergerät, das über eine Leitung mit dem Ultraschallhandstück verbunden ist. In oder an der Leitung ist ein Speicher angeordnet, der der Identifikation des Handstücks dient.

Aus der US 2006/0255467 A1 ist ein Speicherbaustein bekannt, der lediglich zwei Kontaktflächen zur Kommunikation mit einer äußeren Peripherie aufweist. Die Kontaktflächen sind rechteckig gestaltet und unterschiedlich lang.

Weiterer Stand der Technik wird durch die DE 100 57 585 A1, die EP 2 486 885 sowie DE 20 2012 013 520 U1 gebildet.

Es ist durchaus möglich, verschiedene Pumpeneinheiten in ein und dasselbe Pumpenantriebsgerät einzusetzen. Den verschiedenen Pumpeneinheiten können dabei unterschiedliche medizinische Prozeduren zugeordnet sein, wie beispielsweise Medikamenten-Langzeitdosierung oder auch Versorgung eines Instruments bei einem chirurgischen Eingriff.

Es ist Aufgabe der Erfindung, ein Konzept zu schaffen, bei dem das aus Pumpenantriebsgerät und Pumpe bestehende System höheren Anforderungen an Patientensicherheit und Behandlungsqualität gewachsen ist.

Diese Aufgabe wird mit dem Pumpenantriebsgerät nach Anspruch 1 gelöst. Zur Lösung der genannten Aufgabe kann auch die Pumpe nach Anspruch 11 beitragen.

Das erfindungsgemäße Pumpenantriebsgerät eignet sich insbesondere zum Antrieb von Kolbenpumpen mit mindestens einem, vorzugsweise aber zwei oder auch mehreren Pumpzylindern, in denen jeweils ein Kolben gegen den Zylinder abgedichtet, jedoch linear beweglich angeordnet ist. Bei der Pumpe kann es sich um eine Pumpe handeln, die einen oder mehrere mit einem medizinischen Fluid vorbefüllten Behälter aufweist, dessen Inhalt mit dem Kolben in einem Hub abgegeben wird. Eine solche Pumpe weist dann zumindest für den betreffenden Zylinder keinen Ansaugstutzen, sondern lediglich einen Auslass auf. Die Pumpe kann aber auch einen oder mehrere Zylinder aufweisen, die durch hin und her gehende Kolbenbewegung ein Fluid, insbesondere eine Flüssigkeit ansaugen und ausstoßen können. Den Zylindern solcher Pumpen können Einlässe und Auslässe zugeordnet sein. Steuerventile (Rückschlagventile) können in der Pumpe angeordnet sein. Alternativ können die Steuerventile auch Teil des Pumpenantriebsgeräts sein. In diesem Fall weisen die Zylinder der Pumpe jeweils nur einen Anschluss auf, der sowohl als Einlass als auch als Auslass dient.

Das medizinische Pumpenantriebsgerät weist ein Aufnahmefach für eine Pumpe auf. In oder an dem Aufnahmefach ist ein Kontaktsatz angeordnet, der wenigstens vier im Viereck angeordnete Kontakte umfasst. Diese dienen zur Kontaktgabe an Kontaktflächen, die von der medizinischen Pumpe präsentiert werden, d.h. an einer Stelle derselben angeordnet sind, an der sie mit den Kontakten in Berührung stehen, wenn die Pumpe in das Antriebsgerät eingesetzt ist. Dabei kommen wenigstens einige der Kontakte des Kontaktsatzes mit den Kontaktflächen in Verbindung. Der Kontaktsatz kann auch mehr, z.B. fünf, sechs, sieben, acht oder mehrere Kontakte aufweisen. Der Kontaktsatz ist vorzugsweise an einer Stelle des Aufnahmefachs angeordnet, mit der die Pumpe erst in Anlage gelangt, wenn sie vollständig in das Aufnahmefach eingesetzt ist.

Das Pumpenantriebsgerät weist eine Logikeinheit auf, die dazu eingerichtet ist, zu erfassen, ob, und wenn ja, welche Kontakte des Kontaktsatzes durch Kontaktflächen der Pumpe miteinander elektrisch kurzgeschlossen sind. Die Logikeinheit kann dazu eingerichtet sein, den Kontakten des Kontaktsatzes, abhängig davon, welche Kontakte elektrisch miteinander kurzgeschlossen, d.h. verbunden sind, elektrisehe Funktionen zuzuordnen. Das ist insbesondere dann sinnvoll, wenn die beteiligten Kontakte des Kontaktsatzes in einem Quadrat angeordnet sind.

Die Logikeinheit kann dazu eingerichtet sein, den Kontakten die Funktion der Kommunikation mit einem Speicherbaustein zuzuweisen, wenn nur zwei der wenigstens vier Kontakte miteinander elektrisch verbunden sind. In diesem Fall hat die Logikeinheit nur ein kurzgeschlossenes Kontaktpaar festgestellt. Der Funktionsblock kann dazu eingerichtet sein, den Kontakten eine Funktion, zum Beispiel "Masse" oder "Datenleitung" zuzuweisen, wenn nur ein einziges elektrisch verbundenes Kontaktpaar erfasst wird. Dies ist insbesondere sinnvoll, wenn der Kontaktsatz lediglich vier Kontakte aufweist. Umfasst der Kontaktsatz mehr als vier Kontakte, bspw. sechs Kontakte, kann die Logikeinheit weiter dazu eingerichtet sein, vier im Quadrat angeordnete nach obigem Schema auszuwerten und zusätzlich die beiden zusätzlichen Kontakte darauf zu überprüfen, ob sie untereinander oder mit anderen Kontakten verbunden sind. Die Logikeinheit kann darauf eingerichtet sein, den kontaktierten Baustein als Speicherbaustein zu behandeln, wenn die beiden zusätzlichen Kontakte hochohmig, d.h. nicht untereinander und mit keinem anderen Kontakt verbunden sind.

Die Logikeinheit kann außerdem dazu eingerichtet sein, festzustellen, dass die Pumpe keinen Speicherbaustein, sondern einen Kennkontaktträger enthält. Hat der Kontaktträger vier im Quadrat angeordnete Kontakte, kann sie dazu eingerichtet sein, zu erfassen, dass zwei Kontaktpaare mit jeweils zwei untereinander elektrisch verbundenen Kontakten vorhanden sind. Die Logikeinheit kann dazu eingerichtet sein, in diesem Fall verschiedene Pumpentypen zu unterscheiden. Sie kann weiter dazu eingerichtet sein, ein pumpentypkennzeichnendes Signal zu erzeugen und weiterzugeben, und zwar abhängig davon, welche der vier Kontakte jeweils zu Kontaktpaaren verbunden sind. Hat der Kontaktträger zusätzlich zu diesen vier Kontakten ein, zwei oder mehrere weitere Kontakte, können anhand unterschiedlicher, durch Kontaktflächen des Kennkontaktträgers gebildete Verbindungsmuster eine große Anzahl verschiedener Pumpentypen unterschieden werden. Außerdem kann der Kennkontaktträger auch einen oder mehrere elektronische Bausteine, z.B. einen Mikrocontroller, einen Speicher, einen RFID-Chip oder einen sonstigen Kommunikationsbaustein aufweisen. Die Logikeinheit kann dazu eingerichtet sein, das Vorhandensein eines solchen Bausteins durch ein vorbestimmtes, durch die Kontaktflächen des Kennkontaktträgers gebildetes Muster elektrischer Verbindungen zu erkennen und den einzelnen Kontakten dann zum Betrieb und zur Versorgung des Bausteins geeignete Funktionen zuzuweisen.

Mit dem erfindungsgemäßen Konzept lassen sich die Funktionen des Pumpenantriebsgeräts und der zugehörigen Pumpen stark erweitern. Zum Beispiel kann die Pumpe einen Speicherbaustein enthalten, der nach dem Einsetzen der Pumpe in das Pumpenantriebsgerät auslesbar und/oder beschreibbar ist. Ist anstelle des oder zusätzlich zu dem Speicherbaustein ein Mikrocontroller vorhanden, können weitere Funktionen realisiert werden.

Der Speicherbaustein kann über den Kontaktsatz mit der Geräteelektronik verbunden werden. Dadurch können beispielsweise Pumpen vom Originalhersteller von Pumpen unterschieden werden, die von Drittherstellern stammen. Sind die Pumpen mit Medikamenten vorbefüllt, lässt sich dadurch die Patientensicherheit erhöhen. Außerdem kann der Speicherbaustein eine digitale, vom Pumpenantriebsgerät auslesbare Seriennummer enthalten und auf diese Weise den Pumpentyp signalisieren. Außerdem können mit dem erfindungsgemäßen System viele weitere Funktionen realisiert werden, wie beispielsweise das Registrieren und Nachverfolgen der Betriebsstunden der Pumpe. Weiter gibt es auch die Möglichkeit, dass feststellbar ist, ob, wie oft und in welchen Geräten die Pumpe bereits verwendet wurde.

Ist anstelle des Speicherbausteins in dem entsprechenden Sitz ein Kennkontaktträger angeordnet, kann damit ebenfalls eine Unterscheidung von wenigstens zwei Pumpentypen vorgenommen werden. In jedem Fall aber kann anhand der erfassten Kontaktierung der Kontaktflächen der Pumpe der korrekte Einsatz derselben in dem Pumpenantriebsgerät erfasst werden.

Gegenstand der Erfindung ist auch die Pumpe, die in das Pumpenantriebsgerät einsetzbar ist und die einen Sitz zur Aufnahme eines Kontaktträgers aufweist. Der Kontaktträger kann entweder der oben genannte Speicherbaustein oder der oben genannte Kennkontaktträger sein. Der Sitz ist vorzugsweise mit einer Rasteinrichtung versehen, die ein leichtes Einführen des Kontaktträgers in den Sitz gestattet und den Kontaktträger danach unverlierbar in dem Sitz hält.

Weitere Vorteile ergeben sich aus der Zeichnung sowie der nachfolgenden Beschreibung von Ausführungsformen der Erfindung sowie Unteransprüchen.

Es zeigen:
Figur 1 ein Pumpenantriebsgerät mit darin eingesetzter Pumpe,
Figur 2 die Pumpe nach Figur 1, in schematisierter Perspektivdarstellung,
Figur 3 ein Speicherbaustein sowie zwei alternative Kontaktträger zur Befestigung an der Pumpe nach Figur 2,
Figur 4 einen an dem Gehäuse der Pumpe nach Figur 2 ausgebildeten Sitz zur Aufnahme des Speicherbausteins oder eines der Kontaktträger nach Figur 3,
Figur 5 einen an dem Gerät nach Figur 1 vorgesehenen Kontaktsatz mit vier Federkontakten, angeschlossen an eine Logikeinheit zur Auswertung der detektieren Kontakte,
Figur 6 bis 11 verschiedene Kontaktflächenpositionen und -Formen für einen Kontaktsatz mit vier Kontakten sowie die Kontaktgabe der Kontaktflächen zu Kontakten des Kontaktsatzes, jeweils in schematischer Darstellung,
Figur 12 einen Kontaktsatz mit sechs Kontakten in Draufsicht,
Figur 13 bis 17 verschiedene Kontaktflächenpositionen und -Formen sowie deren Kontaktgabe zu Kontakten des Kontaktsatzes, jeweils in schematischer Darstellung.

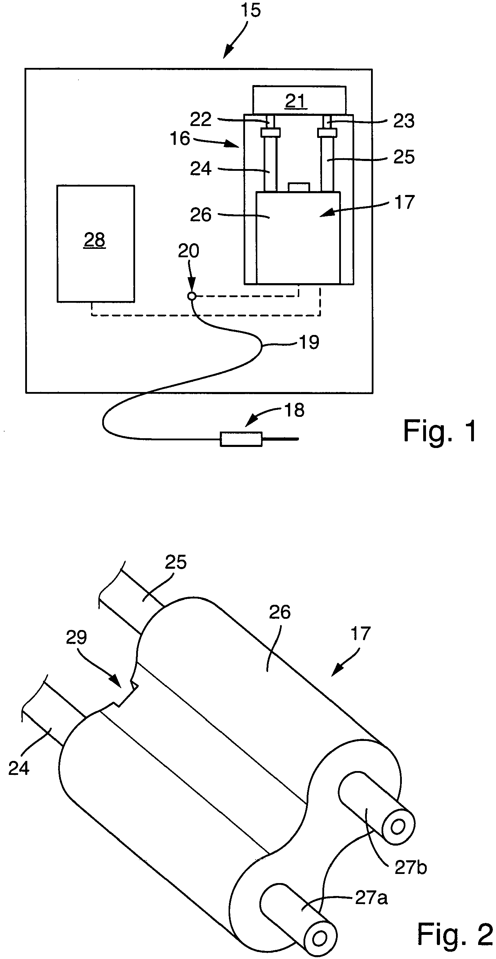

Figur 1 veranschaulicht ein medizinisches Pumpenantriebsgerät 15 mit einem Aufnahmefach 16, in das eine Pumpe 17 eingesetzt ist. Die Pumpe 17 ist vorzugsweise als Kolbenpumpe ausgebildet und dient dazu, ein medizinisches Instrument 18 mit einer Flüssigkeit zu versorgen. Das Instrument 18 ist dazu über eine Leitung 19 an das Pumpenantriebsgerät 15 oder an einem entsprechenden Anschluss 20 angeschlossen. Der Anschluss 20 steht in Fluidverbindung mit der Pumpe 17, die in eine entsprechende in dem Aufnahmefach 16 vorgesehene Fassung eingesetzt ist. Der Anschluss 20 kann alternativ auch direkt an der Pumpe 17 vorgesehen sein.

Je nach Anwendungsfall kann das Instrument 18 ein chirurgisches Instrument, eine Kanüle oder irgendeine andere Einrichtung sein, die die von der Pumpe 17 geförderte Flüssigkeit einem Patienten zuführt. Die Flüssigkeit kann der Durchführung oder Unterstützung eines chirurgischen Eingriffs dienen oder dem Patienten, z.B. intravaskulär, als Medikament zugeführt werden.

Das Pumpenantriebsgerät 15 enthält eine in Figur 1 lediglich schematisch angedeutete Antriebseinrichtung 21, mit einem oder mehreren Antriebsstößeln 22, 23, die von der Antriebseinrichtung 21 gleichlaufend oder alternativ gegenlaufend linear angetrieben sind. Sie dienen zur Betätigung, d.h. linearen Verschiebung von zueinander parallelen Pumpenstößeln 24, 25, mit denen Kolben in einem entsprechenden Zylinderblock 26 der Pumpe 17 in jeweiliger Stößel-Längsrichtung bewegbar sind. Durch die Bewegung der Kolben der Pumpe 17 wird in den Zylindern des Zylinderblocks 26 vorhandenes Fluid an Pumpenausgängen 27a, 27b abgegeben, die aus Figur 2 ersichtlich sind.

Die Pumpenausgänge 27a, 27b können direkt mit dem Anschluss 20 verbunden sein. In diesem Fall ist die Pumpe 17 mit medizinischem Fluid vorbefüllt und dient lediglich der Ausgabe desselben. Es ist jedoch auch möglich, die Pumpe 17 dazu zu nutzen, aus einem in Figur 1 lediglich schematisch veranschaulichten Vorrat 28 ein Fluid, beispielsweise Natriumchloridlösung oder eine andere Flüssigkeit, anzusaugen und über den Anschluss 20 abzugeben. Es ist dabei möglich, dass lediglich einer der beiden Stößel 24, 25 zum wiederholten Ansaugen und Abgeben des Fluids an das Instrument 18 dient, während der andere Stößel der Zudosierung einer zuvor in die Pumpe 17 eingefüllten medizinischen Flüssigkeit dient. Weiter alternativ können auch beide Pumpenstößel 24, 25 hin und her gehend bewegt werden, um abwechselnd Fluid, zum Beispiel aus dem Vorrat 28, anzusaugen und an das Instrument 18 abzugeben, wodurch ein ununterbrochener Fluidstrom erzeugbar ist. Die entsprechenden Ventile zum Steuern des Ansaug- und Ausstoßvorgangs an jeder einzelnen Pumpeneinheit der Pumpe 17, die von den Pumpenstößeln 24, 25 bedient werden, können in der Pumpe 17 oder alternativ auch in dem Pumpenantriebsgerät 15 angeordnet sein. Es ist auch möglich, die Ein- und Auslassventile der Pumpe so anzuordnen und mit zwei verschiedenen Flüssigkeitsreservoiren zu verbinden, dass zwei unterschiedliche Flüssigkeiten aus den zwei Reservoiren angesaugt, in der Pumpe gemischt und dann als Mischung ausgetragen werden.

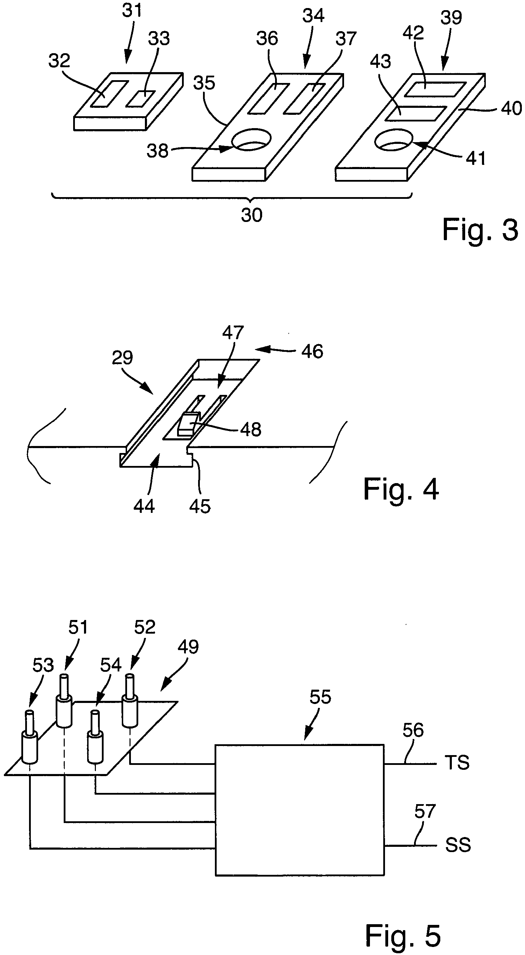

Die Pumpe 17 weist zum Beispiel an ihrem Zylinderblock 26 einen Sitz 29 zur Aufnahme eines Kontaktträgers 30 auf. Der Sitz 29 ist vorzugsweise an dem von den Pumpenausgängen 27, 28 weg liegenden Ende des Zylinderblocks 26, zum Beispiel zwischen den Pumpenstößeln 24, 25, angeordnet. Entsprechende, von dem Sitz 29 aufnehmbare Kontaktträger 30 sind in Figur 3 veranschaulicht, während der Sitz 29 detaillierter in Figur 4 dargestellt ist.

Der Kontaktträger 30 kann zum Beispiel ein Speicherchip 31 sein, an dessen flachen, plattenartigen, im Umriss quadratischen Gehäuse zwei Kontaktflächen 32, 33 angeordnet sind. Die Kontaktflächen 32, 33 sind zum Beispiel rechteckig ausgebildet und entlang ihrer Längsrichtung parallel zueinander orientiert. Die beiden Kontaktflächen 32, 33 können dabei in Kontaktlängsrichtung jeweils eine unterschiedliche Länge aufweisen. Beispielsweise ist der Speicherchip 31 dazu konzipiert, nach dem One-Wire-Protokoll zu arbeiten, d.h. er nutzt eine der Kontaktflächen 32, 33, beispielsweise die Kontaktfläche 32, als Bezugsmasse und die jeweils andere Kontaktfläche, hier die Kontaktfläche 33, als Datenleitung. Die Kontaktflächen 32, 33 sind vorzugsweise im Wesentlichen ebene Flächen, die in einer gemeinsamen Ebene angeordnet sind. In dem Gehäuse 31 ist ein elektronischer Baustein beispielsweise in Gestalt eines Speicherchips angeordnet, der elektrisch mit den Kontaktflächen 32, 33 verbunden ist.

In Figur 3 ist außerdem ein Kennkontaktträger 34 veranschaulicht, der einen elektrisch nicht leitenden in Draufsicht rechteckförmigen Grundkörper 35 aufweist, auf dem Kontaktflächen 36, 37 angeordnet sind. Diese weisen vorzugsweise gleich große rechteckige Umrisse auf und sind mit ihren langen Kanten parallel zueinander orientiert. Die langen Kanten der beiden Kontaktflächen 36, 37 sind dabei parallel zu den langen Seiten des in Draufsicht rechteckförmigen Grundkörpers 35 orientiert. Die Kontaktflächen 36, 37 kennzeichnen mit ihrer Form und Anordnung die Pumpe 17 und sind deswegen Kennkontaktflächen. Der Umstand, dass beide Kontaktflächen 36, 37 jeweils zwei Kontakte des Kontaktsatzes 49 überspannen und im Einsatz elektrisch miteinander verbinden, ist ein Kennzeichen dafür, dass der Kontaktträger 30 kein aktives elektronisches Bauelement enthält.

Der Grundkörper 35 weist außerdem eine Öffnung 38 auf, die zur Arretierung des Kennkontaktträgers 34 in dem Sitz 29 dient. Die Öffnung 38 kann auch als Handlingöffnung genutzt werden, die die Handhabung des Kennkontaktträgers durch einen Bestückungsautomaten erleichtert. Jedenfalls aber lässt sich der passive Kennkontakträger 34 auf Grund seiner Rechteckform und der leichten, durch die Öffnung 38 gegebenen Orientierbarkeit ohne Schwierigkeiten in gewünschter Orientierung in den Sitz 29 einsetzen. Im Gegensatz dazu ist die Orientierung des Speicherchips 31 vierfach unbestimmt.

Ein alternativer Kennkontaktträger 39 ist in Figur 3 rechts veranschaulicht. Während der Grundkörper 40 desselben mit dem Grundkörper 35 übereinstimmt und wie dieser eine Öffnung 41 aufweist sind seine Kontaktflächen 42, 43 quer zu den langen Seiten des Grundkörpers 40 orientiert. Die Kontaktflächen 42, 43 sind wiederum rechteckig und untereinander gleichgroß ausgebildet. Ergänzend gelten die für den Kennkontaktträger 34 gegebenen Ausführungen für den Kennkontaktträger 39 entsprechend.

Jeder der Kontaktträger 30 nach Figur 3 kann in dem Sitz 29 angeordnet werden. Der Sitz 29 weist dazu einen Einschubkanal 44 auf, der an seiner Stirnseite eine Einführöffnung 45 aufweist. An seiner zur Einführrichtung parallelen oberen Flachseite ist der Einführkanal 44 offen, so dass ein in den Einführkanal 44 eingeschobener Kontaktträger 30 mit seinen jeweiligen Kontaktflächen 32,33; 36,37 oder 42,43 zugänglich bleibt. Zur Arretierung des jeweiligen Kontaktträgers 30 in den Einführkanal 44 des Sitzes 29 ist ein Rastmittel 46, beispielsweise in Gestalt einer Rastzunge 47 vorgesehen. Diese ist an dem Boden angeordnet, der den Einführkanal 44 begrenzt. Die Rastzunge 47 weist eine Rastnase 48 auf, um den Kontaktträger 30 in dem Einführkanal und somit in dem Sitz 29 zu sichern.

Auf Seiten des Pumpenantriebsgeräts 15 ist an einer dem Sitz 29 gegenüberliegenden Stelle ein Kontaktsatz 49 angeordnet, der mindestens vier Kontakte 51, 52, 53, 54 aufweist. Diese sind zum Beispiel als stiftartige Federkontakte ausgeführt und an den Ecken eines gedachten Quadrats parallel zueinander von einem Träger aufragend angeordnet. Sie sind mit den Kontaktflächen 32, 33; 36, 37; 42, 43 in elektrischen Kontakt überführt, wenn die Pumpe 17 ordnungsgemäß mit einem der Kontaktträger 30 versehen und in das Pumpenantriebsgerät 15 korrekt eingesetzt ist.

Die Kontakte 51 bis 54 sind mit einer Logikeinheit 55 verbunden, die dazu eingerichtet ist, anhand der sich zwischen den Kontakten 51 bis 54 einstellenden, von den Kontaktflächen 32,33; 36,37; 42,43 gebildeten elektrischen Verbindungen festzustellen, um welchen Pumpentyp es sich bei der Pumpe 17 handelt und/oder ob von der Pumpe 17 bzw. deren Speicherchip 31 herrührende Daten verfügbar sind. Die Logikeinheit 55 weist dabei einen Ausgang 56 für ein Pumpentyp kennzeichnendes Signal TS und einen Ausgang 57 für die Ausgabe eines Speichersignals SS auf. Es wird darauf hingewiesen, dass die Ausgänge 56, 57 hardwaremäßige Ausgänge oder auch Programmausgänge sein können. Die Ausgänge können auch zu einem Ausgang zusammengefasst sein, wenn die Signale TS und SS unterscheidbar sind. Die Logikeinheit 55 kann als hardwaretechnischer Baustein oder auch als Programmbaustein oder Element eines Programms realisiert sein, das auf einem programmierbaren Baustein läuft.

Das insoweit beschriebene Pumpenantriebsgerät arbeitet in Verbindung mit der beschriebenen Pumpe 17 wie folgt:

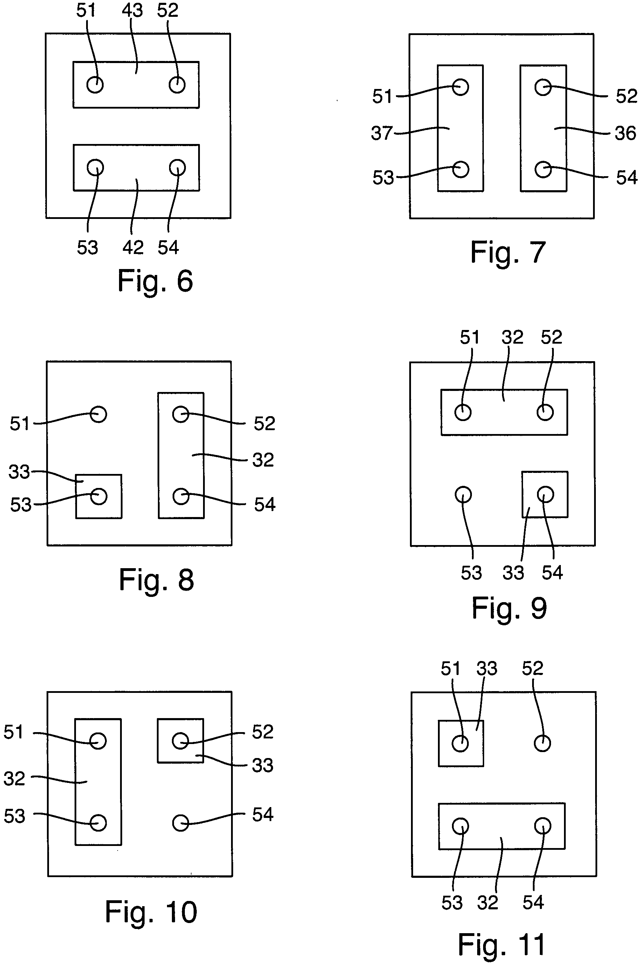

Zur Inbetriebnahme des Pumpenantriebsgeräts 15 wird die Pumpe 17 in das Aufnahmefach 16 eingesetzt. Zur weiteren Veranschaulichung der Funktion wird davon ausgegangen, dass die Pumpe 17 als Kontaktträger 30 den Kennkontaktträger 39 aufweist. Dieser kommt mit den Kontakten 51 bis 54 wie in Figur 6 veranschaulicht in Berührung. Die Kontaktfläche 42 schließt die Kontakte 53, 54 elektrisch miteinander kurz. Die Kontaktfläche 43 schließt die Kontakte 51, 52 miteinander kurz. Die Logikeinheit 55 testet nun die zwischen den Kontakten 51 bis 54 vorhandenen elektrischen Verbindungen und erkennt das Kontaktpaar 51/52 als miteinander kurzgeschlossen. Ebenso findet sie das Kontaktpaar 53/54 als miteinander kurzgeschlossen vor. Diese Kontaktpaarung ist einem ersten Pumpentyp vorbehalten und zugeordnet. Die Logikeinheit 55 kann deswegen an dem Ausgang 56 ein den Pumpentyp kennzeichnendes Signal TS abgeben. Gleichzeitig kann die korrekte Abgabe dieses Signals als Kennzeichen dafür dienen und genutzt werden, dass die Pumpe 17 korrekt in das Aufnahmefach 16 eingesetzt ist.

Figur 7 veranschaulicht die Situation, wenn eine Pumpe 17 mit dem Kennkontaktträger 34 in das Aufnahmefach 16 eingesetzt worden ist. Die Kontaktfläche 36 überbrückt elektrisch die Kontakte 52 und 54, während die Kontaktfläche 37 die Kontakte 51 und 53 überbrückt. Anhand der Kontaktpaare 51/53, 52/54 erkennt die Logikeinheit 55 den zugeordneten Pumpentyp (zweiter Pumpentyp) und gibt an dem Ausgang 56 ein entsprechendes Pumpentypkennzeichnungssignal TS aus.

Prinzipiell sind auch weitere Pumpentypkennzeichnungen möglich. Beispielsweise können die Kontaktflächen 42 und 43 untereinander elektrisch verbunden sein. Damit ist ein dritter Pumpentyp erkennbar, wenn alle Kontakte 51 bis 54 untereinander elektrisch verbunden sind. (Entsprechendes gilt für die Kontaktflächen 36 und 37). Bei komplizierterem Aufbau des Kennkontaktträgers können auch die diagonal gegenüberliegenden Kontakte paarweise verbunden werden, sodass ein vierter Pumpentyp unterscheidbar und erkennbar ist.

Prinzipiell sind auch Ausführungsformen mit mehr als vier Kontaktstiften und entsprechend mehr als zwei Kontaktflächen denkbar. Die Kontaktflächen können auch nichtrechteckigförmig, bspw. L-förmig, ausgebildet sein. Hierdurch lässt sich eine noch höhere Anzahl an Pumpentypen codieren.

Die Figuren 8 bis 11 veranschaulichen die Situation bei einer Pumpe 17, in deren Sitz 29 ein Speicherchip 31 angeordnet ist. Weil dieser einen quadratischen Umriss hat, kann er prinzipiell in vier verschiedenen Orientierungen in den Sitz 29 eingeführt sein. Die Logikeinheit 55 ist darauf eingerichtet, die Einbaulage zu erkennen und unabhängig von ihr die korrekte Funktion sicher zu stellen.

Bei der Orientierung nach Figur 8 überbrückt die Kontaktfläche 32 die Kontakte 52 und 54 während der Kontakt 51 unkontaktiert bleibt. Die Logikeinheit 55 ist dazu eingerichtet, zu erkennen, dass die Kontakte 52 und 54 als Bezugsmassekontakte zu behandeln sind. Sie ist weiter dazu eingerichtet, den Kontakt 53 als Datenkontakt zu behandeln. Auf diese Weise kann die Logikeinheit 55 mit dem Speicherchip 31 kommunizieren und an dem Ausgang 57 ein entsprechendes Speichersignal SS ausgeben. So kann das Signal zum Beispiel zur Identifikation der Pumpe 17 oder ihres Inhalts genutzt werden. Es ist auch möglich, über nicht weiter veranschaulichte Mittel Daten in den Speicherchip 31 einzuschreiben, wenn dieser als beschreibbares Speichermedium ausgebildet ist.

Figur 9 veranschaulicht die Situation, wenn die Kontaktfläche 32 die Kontakte 51 und 52 überbrückt. In diesem Fall erkennt die Logikeinheit 55, dass diese beiden Kontakte 51, 52 als Bezugsmassekontakte zu behandeln sind, während der Kontakt 54 der Datenkontakt ist. Entsprechend sind in Figur 10 die Kontakte 51, 53 die Bezugsmasse, und der Kontakt 52 ist der Datenkontakt. Hat der Speicherchip 31 die Ausrichtung nach Figur 11, interpretiert die Logikeinheit die Kontakte 53, 54 als Bezugsmassekontakte und den Kontakt 51 als Datenkontakt. Wie ersichtlich, sind die Funktion des Speicherchips 31 und die Kommunikation mit einer entsprechenden Auswerteeinrichtung in dem Pumpenantriebsgerät unabhängig von der Ausrichtung des Speicherchips 31 in dem Sitz 29. Bei der Bestückung der Pumpe 17 während ihrer Herstellung muss keine Rücksicht auf die Ausrichtung des Speicherchips 31 genommen werden. Es genügt, wenn seine Kontaktflächen 32, 33 zugänglich sind. Die Funktion der Kontaktflächen 32, 33 wird immer anhand dessen erkannt, welcher der Kontakte 51 bis 54 unkontaktiert bleibt und/oder welches Kontaktpaar 52/54; 51/52; 51/53; 53/54 elektrisch kurzgeschlossen ist.

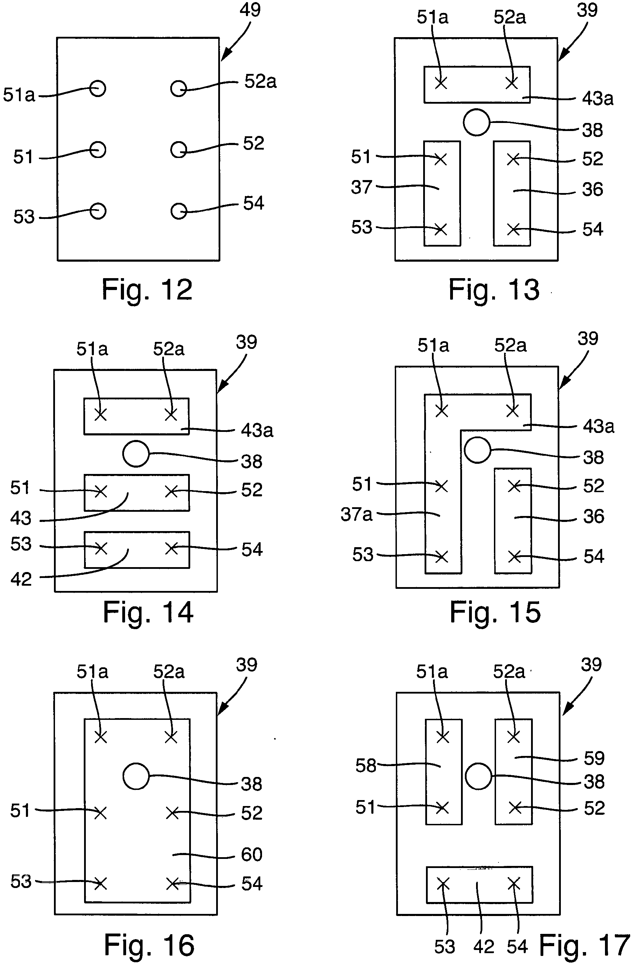

Zusätzlich zu den vier im Quadrat angeordneten Kontakten 51, 52, 53, 54 können, wie Figur 12 veranschaulicht, weitere Kontakte 51a, 52a vorgesehen sein. Beispielsweise können diese wie veranschaulicht in zwei zueinander parallelen Dreierreihen angeordnet sein, wobei die vier Kontakte 51, 52, 53, 54 wiederum im Quadrat angeordnet sind oder sein können.

Die Figuren 13 bis 17 veranschaulichen einige der hier möglichen Kontaktkonfigurationen zur Kennzeichnung verschiedener Pumpentypen, Pumpenverwendungszwecke, Pumpenfüllungen oder auch mit Eignung zur Anordnung weiterer elektronischer Bauelemente in oder an dem jeweiligen Kennkontaktträger 39.

Die Logikeinheit 55 ist an alle sechs Kontakte 51, 51a, 52, 52a, 53, 54 angeschlossen und wertet die zwischen den Kontakten gemessenen Widerstände aus. Dazu ist sie dazu eingerichtet, die Überbrückung bzw. die Widerstände zwischen den Kontakten 51, 52, 53, 54, wie vorstehend und im Zusammenhang mit Figur 5 beschrieben, auszuwerten. Zusätzlich kann die Logikeinheit 55 darauf eingerichtet sein, die Kontakte 51a, 52a dahingehend zu überprüfen, ob diese untereinander oder mit einem der anderen Kontakte 51 bis 54 verbunden sind. Ist dies nicht der Fall, kann die Logikeinheit 55 daraus schließen, dass in dem Sitz 29 ein Speicherbaustein in einer der Ausrichtungen nach Figur 8, 9, 10 oder 11 angeordnet ist. Wird jedoch an den Kontakten 51a, 52a irgendeine elektrische Verbindung zu einem der anderen Kontakte festgestellt, kann die Logikeinheit 55 daraus schließen, dass ein Kennkontaktträger 39 nach Figur 13 bis 17 in den Sitz 29 eingesetzt worden ist. Der Kennkontaktträger 39 nach Figur 13 weist zwei Kontaktflächen 36, 37 ähnlich wie der Kennkontaktträger nach Figur 7 auf. Zusätzlich ist eine Kontaktfläche 43a vorgesehen, die wenn die Pumpe 17 in das Aufnahmefach 16 eingesetzt ist, die Kontakte 51a, 52a miteinander elektrisch verbindet. Das entstehende Verbindungsmuster (51, 53; 52, 54; 51a, 52a) kann zur Kennzeichnung eines bestimmten Pumpentyps oder einer bestimmten Vorbefüllung der Pumpe oder einer der Pumpe zugeordneten Betriebsweise genutzt werden. Die Logikeinheit 55 kann darauf eingerichtet sein, diese zu erfassen, zu identifizieren und ein entsprechendes Signal TS abzugeben.

Außerdem können die Kontaktpaare 51a/52a, 51/53, 52/54 auch als Massekontakt, Betriebsspannungskontakt und Datenkontakt für einen Eindrahtmikrocontroller genutzt werden, der auf den Kennkontaktträger 39 untergebracht sein kann, um mit der Steuerung des Pumpenantriebsgeräts, insbesondere der Logikeinheit 55 zu kommunizieren. Gleiches gilt für die Kennkontaktträger 39 nach Figur 14 und Figur 17, bei denen jeweils drei untereinander durch Kennkontaktflächen verbundene Kontaktpaare vorhanden sind. Bei der Ausführungsform des Kennkontaktträgers 39 nach Figur 14 verbinden die Kontaktflächen 42, 43 und 43a die Kontaktpaare 53/54, 51/52, 51a/52a. Wiederum kann diese Kontaktpaarung als Kennzeichen für einen bestimmten Pumpentyp, für eine bestimme Pumpenfüllung oder auch zum Betrieb eines Mikrocontrollers auf dem Kennkontaktträger 39 genutzt werden.

Entsprechendes gilt für den Kennkontaktträger nach Figur 17, bei dem die Kontaktfläche 42 die Kontakte 53, 54 verbindet während die Kontaktfläche 58 die Kontakte 51a, 51 und die Kontaktfläche 59, die Kontakte 52a, 52 miteinander elektrisch verbindet. Zur Nutzung der Kontakte 51, 51a, 52, 52, 53, 54 gelten die Ausführungen zu Figur 13 und 14 entsprechend.

Eine weiter abgewandelte Ausführungsform zeigt Figur 15. Dieser Kennkontaktträger weist eine L-förmig ausgebildete Kennkontaktfläche 37a-43a auf, die die Kontakte 51, 51a, 52a, 53 miteinander verbindet. Die Kontaktfläche 36 überbrückt die Kontakte 52, 54. Auch dieses Kontaktgabemuster kann wiederum für einen bestimmten Pumpentyp, eine Pumpenfüllung, eine Pumpenbetriebsweise oder dergleichen stehen, was von der Logikeinheit 55 erkannt wird, sodass dies an ihrem Ausgang 56 ein entsprechendes Pumpentypkennzeichnendes Signal TS ausgibt.

Es kann wie Figur 16 veranschaulicht auch eine Kontaktfläche 60 vorgesehen sein, die alle Kontakte 51, 51a, 52, 52a, 53, 54 miteinander verbindet, um wiederum einen bestimmten Pumpentyp, eine Pumpenfüllung, eine Pumpenverwendungsweise, eine Pumpenbetriebsweise oder dergleichen zu kennzeichnen.

Alle Kennkontaktträgern 39 ist gemeinsam, dass sie unabhängig von ihrer sonstigen Funktion dienen können, das korrekte Einsetzen der Pumpe 17 in das Pumpenaufnahmefach 16 zu kennzeichnen. Entspricht die Kontaktgabe keinem erwartbaren Kontaktmuster oder ist keine Kontaktgabe vorhanden, erkennt die Logikeinheit 55 dies und sperrt den Betrieb des Pumpenantriebsgeräts so lange bis ein gültiges Kontaktgabemuster erkannt wird.

Das erfindungsgemäße Konzept richtet sich auf eine Pumpenantriebseinheit 15 und die zugehörige Pumpe 17, die einen Sitz 29 aufweist, der alternativ zur Aufnahme eines Speicherchips 31 oder von Kennkontaktträgern 34, 39 genutzt werden kann. Die Pumpenantriebseinheit weist mindestens vier elektrische Kontakte auf, die mit dem Kontaktträger 30 (entweder Speicherchip 31 oder Kennkontaktträger 34, 39) bzw. deren Kontaktflächen 32,33; 36,37; 42,43 in Kontakt überführbar sind. Eine in dem Pumpenantriebsgerät 15 vorgesehene Logikeinheit 55 kann dazu dienen, zu unterscheiden, ob als Kontaktträger 30 ein Speicherchip 31 oder ein Kennkontaktträger 34, 39 angeordnet ist. Detektiert die Logikeinheit 55 einen Speicherchip 31, tritt sie mit diesem in elektrische Kommunikation. Detektiert sie hingegen einen Kennkontaktträger 34, 39 bestimmt sie anhand der Ausrichtung seiner Kontaktflächen 32,33; 36,37; 42,43 dessen Typ und gibt ein entsprechendes pumpentypkennzeichnendes Signal TS ab.

Das erfindungsgemäße Konzept eröffnet einen weiten Bereich von zusätzlichen Nutzungsmöglichkeiten grundsätzlich bekannter Pumpensysteme mit Nutzen für die Erhöhung der Patientensicherheit und Erleichterung der Bedienbarkeit.

### Bezugszeichen

- 15: Pumpenantriebsgerät
- 16: Aufnahmefach
- 17: Pumpe
- 18: Instrument
- 19: Leitung
- 20: Anschluss
- 21: Antriebseinrichtung
- 22,23: Antriebsstößel
- 24,25: Pumpenstößel
- 26: Zylinderblock
- 27a,27b: Pumpenausgänge
- 28: Flüssigkeitsvorrat
- 29: Sitz
- 30: Kontaktträger
- 31: Speicherchip
- 32,33: Kontaktflächen
- 34: Kennkontaktträger
- 35: Grundkörper
- 36,37: Kontaktflächen
- 38: Öffnung
- 39: Kennkontaktträger
- 40: Grundkörper
- 41: Öffnung
- 42,43,43a: Kontaktflächen
- 44: Einführkanal
- 45: Einführöffnung
- 46: Rastmittel
- 47: Rastzunge
- 48: Rastnase
- 49: Kontaktsatz
- 51 - 54: Kontakte
- 55: Logikeinheit
- 56,57: Ausgänge
- TS: pumpentypkennzeichnendes Signal
- SS: Speichersignal
- 58 - 60: Kontaktflächen

## Patentansprüche

1. Medizinisches Pumpenantriebsgerät (15), insbesondere zum Antrieb von Kolbenpumpen,
mit einem Aufnahmefach (16) für eine Pumpe (17), wobei in oder an dem Aufnahmefach (16) ein Kontaktsatz (49) angeordnet ist, der wenigstens vier im Viereck angeordnete Kontakte (51 - 54) aufweist,
mit einer Logikeinheit (55), die dazu eingerichtet ist, zu erfassen ob, und wenn ja welche der Kontakte (51 - 54) durch Kontaktflächen (32,33; 36,37; 42,43) der Pumpe (17) miteinander elektrisch kurzgeschlossen sind.

2. Pumpenantriebsgerät nach Anspruch 1, **dadurch gekennzeichnet, dass** dem Aufnahmefach (16) wenigstens ein Antriebsstößel (22) zugeordnet ist, der mit einer Antriebseinrichtung (21) verbunden ist, um eine Stößel-Längsbewegung auszuführen.

3. Pumpenantriebsgerät nach einem der vorstehenden Ansprüche, **dadurch gekennzeichnet, dass** dem Aufnahmefach (16) wenigstens zwei Antriebsstößel (22, 23) zugeordnet sind, die zueinander parallel orientiert und jeweils mit einer Antriebseinrichtung (21) verbunden sind, um unabhängig voneinander Stößel-Längsbewegungen auszuführen.

4. Pumpenantriebsgerät nach einem der vorstehenden Ansprüche, **dadurch gekennzeichnet, dass** jeder Kontakt (51 - 54) als federnder Kontaktstift ausgebildet ist und dass alle Kontaktstifte des Kontaktsatzes (49) zueinander parallel orientiert sind.

5. Pumpenantriebsgerät nach einem der vorstehenden Ansprüche, **dadurch gekennzeichnet, dass** die Logikeinheit (55) dazu eingerichtet ist, den Kontakten (51 - 54) des Kontaktsatzes (49) abhängig davon, welche Kontakte (51 - 54) elektrisch miteinander kurz geschlossen sind, elektrische Funktionen zuzuordnen.

6. Pumpenantriebsgerät nach einem der vorstehenden Ansprüche, **dadurch gekennzeichnet, dass** das Viereck ein Quadrat ist.

7. Pumpenantriebsgerät nach einem der vorstehenden Ansprüche, **dadurch gekennzeichnet, dass** die Logikeinheit dazu eingerichtet ist, den Kontakten (51 - 54) die Funktion der Kommunikation mit einem Speicherbaustein (31) zuzuweisen, wenn nur zwei der wenigstens vier Kontakte (51 - 54) miteinander elektrisch verbunden sind.

8. Pumpenantriebsgerät nach Anspruch 7, **dadurch gekennzeichnet, dass** die Logikeinheit (55) dazu eingerichtet ist, den beiden elektrisch miteinander kurz geschlossenen Kontakten (51/52) gemeinsam eine erste und einem der verbleibenden Kontakte (54) eine zweite Funktion zuzuweisen.

9. Pumpenantriebsgerät nach einem der vorstehenden Ansprüche, **dadurch gekennzeichnet, dass** die Logikeinheit (55) dazu eingerichtet ist, ein pumpentypkennzeichnendes Signal (TS) zu generieren, wenn je zwei der vier Kontakte (51 - 54) jeweils als Kontaktpaare (51/52; 53/54) elektrisch miteinander verbunden sind.

10. Pumpenantriebsgerät nach Anspruch 9, **dadurch gekennzeichnet, dass** das pumpentypkennzeichnende Signal (TS) kennzeichnet, welche der wenigstens vier Kontakte (51 - 54) elektrisch zu Kontaktpaaren (51/52; 53/54) verbunden sind.

11. Medizinische Pumpe zum Einsatz in einem Pumpenantriebsgerät nach Anspruch 1, **dadurch gekennzeichnet, dass** die Pumpe Kontaktflächen (32,33; 36,37; 42,43) aufweist, die an einer Stelle angeordnet sind, an der sie mit wenigstens einigen Kontakten (51 - 54) des Kontaktsatzes (49) in Berührung stehen, wenn die Pumpe (17) in das Pumpenantriebsgerät (15) eingesetzt ist.

12. Pumpe nach Anspruch 11, **dadurch gekennzeichnet, dass** die Pumpe (17) einen Sitz (29) zur Aufnahme eines Speicherbausteins (31) aufweist.

13. Pumpe nach Anspruch 12, **dadurch gekennzeichnet, dass** der Sitz (29) zur alternativen Aufnahme eines Kennkontaktträgers (34, 39) eingerichtet ist.

14. Pumpe nach Anspruch 13, **dadurch gekennzeichnet, dass** der Kennkontaktträger (39) wenigstens einen Elektronikbaustein, insbesondere einen Speicher, einen Mikrocontroller, ein RFID-Chip oder einen sonstigen Kommunikationsbaustein enthält.

15. Pumpe nach Anspruch 14, **dadurch gekennzeichnet, dass** die Kontaktflächen (32,33; 36,37; 42,43) an dem Speicherbaustein (31) oder an dem Kennkontaktträger (34, 39) angeordnet sind.
